# EUROPÄISCHE PATENTSCHRIFT

(11) **EP 0 891 960 B1**
(45) Veröffentlichungstag und Bekanntmachung des Hinweises auf die Patenterteilung: **10.04.2002**
(21) Anmeldenummer: 98111005.9
(22) Anmeldetag: 16.06.1998
(51) Int. Cl.: C07C 37/50, C07C 33/26, C07C 41/28, C07C 43/205

(54) **Verfahren zur Herstellung von Brenzcatechinmonoethern und Brenzcatechinen**
Process for the preparation of pyrocathecol mono ethers and pyrocathecols
Procédé pour la préparation de monoéthers de pyrocatéchine et de pyrocatéchines

(30) Priorität: 15.07.1997 DE 19730308
(43) Veröffentlichungstag der Anmeldung: 20.01.1999
(73) Patentinhaber: BASF AKTIENGESELLSCHAFT, 67056 Ludwigshafen (DE)
(72) Erfinder: Heineke, Daniel, Dr., 67487 Maikammer (DE); Dingerdissen, Uwe, Dr., 64342 Seeheim-Jugenheim (DE); Wulff-Döring, Joachim, Dr., 67227 Frankenthal (DE); Hesse, Michael, Dr., 67549 Worms (DE)

(56) Entgegenhaltungen:
- EP-A- 0 499 055
- DE-A- 2 064 097
- DE-A- 2 130 392
- US-A- 3 819 719

## Beschreibung

Die vorliegende Erfindung betrifft ein neues Verfahren zur Herstellung von Brenzcatechinmonoethern und Brenzcatechinen der allgemeinen Formeln Ia und Ib, 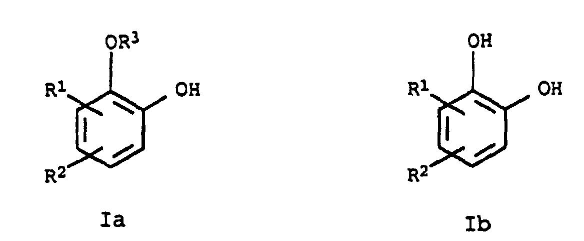 in denen R¹ und R² unabhängig voneinander für Wasserstoff oder C₁-C₈-Kohlenwasserstoffreste stehen und R³ einen C₁-C₈-Kohlenwasserstoffrest bedeutet.

Die Verbindungen Ia und Ib sind bekannt und dienen als Zwischenprodukte für die Synthese von Pharmaprodukten, Riech- und Geschmacksstoffen.

In Ind. Eng. Chem., Prod. Res. Dev., 15(3), 212 (1976) ist die katalytische Hydroxylierung von Phenol mit Wasserstoffperoxid in Gegenwart von Phosphorsäure und Perchlorsäure zu Brenzcatechin und Hydrochinon beschrieben.

Dieses Syntheseverfahren hat jedoch den Nachteil, daß man neben dem Brenzcatechin noch etwa gleiche Mengen Hydrochinon erhält.

Ferner ist aus der DE-A-2 703 077 bekannt, Cyclohexandion-(1,4)-tetramethyldiketal durch katalytische Dehydrierung und Methanolabspaltung in der Flüssigphase zum Hydrochinondimethylether umzusetzen.

DE-A-2 064 097 beschreibt ein Verfahren zur Herstellung von Brenzcatechin und dessen Monoethern durch Dehydrierung entsprechender Cyclohexanonderivate bei Temperaturen von 150-300°C in Gegenwart von Palladium auf Li-Al-Spinell-Trägern.

EP-A-0 499 055 beschreibt ein Verfahren zur Herstellung von Brenzcatechinmonoethern und Brenzcatechin durch Dehydrierung von 2-Hydroxy-cyclohexanondimethylketal an desaktivierten Platinmetallen in Form von Trägerkatalysatoren. Als Träger werden eine Vielzahl von Oxiden, Sulfaten, Silikaten sowie bevorzugt Kohle genannt.

In CA 951742 wird die Umsetzung von 2-Methoxycyclohexanon zu Guajakol an Pd auf Kohle beschrieben.

Sowohl die Ausbeuten und Selektivitäten, aber auch die Katalysatorstandzeiten sind bei den oben beschriebenen Verfahren für eine technische Anwendung zu gering.

Der Erfindung lag somit die Aufgabe zugrunde, Brenzcatechinmonoether Ia und Brenzcatechine Ib auf einfachere und wirtschaftlichere Weise zugänglich zu machen.

Diese Aufgabe wurde gelöst durch ein Verfahren zur Herstellung von Brenzcatechinmonoethern oder Brenzcatechinen der allgemeinen Formel Ia oder Ib 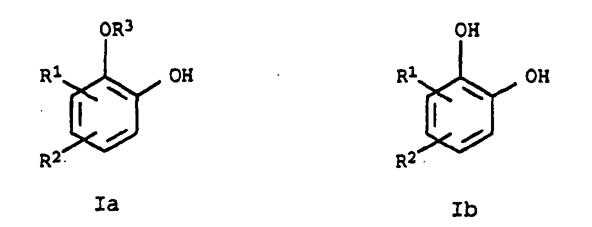 in denen R¹ und R² unabhängig voneinander für Wasserstoff oder C₁-C₈ Kohlenwasserstoffreste stehen und R³ einen C₁-C₈ Kohlenwasserstoffrest bedeutet, dadurch gekennzeichnet, daß man ein 2-Hydroxycyclohexanondialkylketal der allgemeinen Formel II, oder ein 2-Alkoxycyclohexanon der Formel III 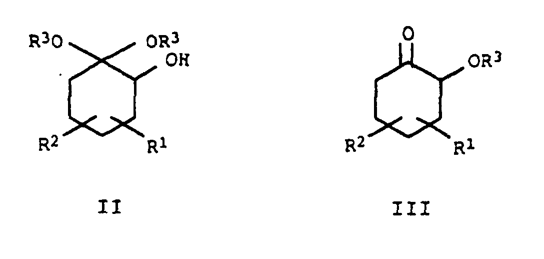
a) zur Gewinnung der Verbindungen Ia in Gegenwart von Palladium auf Zirkonoxid als Katalysator in der Gasphase umsetzt, wobei der Katalysator eine BET-Oberfläche von 10 bis 300 m²/g Katalysator und ein Porenvolumen im Bereich von 0,1 bis 1 ml/g Katalysator besitzt oder
b) zur Gewinnung der Verbindungen Ib die Stufe a) in Gegenwart von Wasser durchführt.

Die Ausgangsverbindungen II und III sind bekannt oder nach bekannten Methoden erhältlich, z.B. durch elektrochemische Oxidation von Cyclohexanon mit Alkanolen in Gegenwart eines Hilfselektrolyten und Wasser und anschließender sauer oder basisch katalysierter Methanolabspaltung (DE-A 4 017 576).

Unter den definitionsgemäßen Verbindungen II und III werden aus wirtschaftlichen Gründen solche bevorzugt, in denen die Reste folgende Bedeutung haben:
- R¹,R²: Wasserstoff;
- R¹-R³: C₁-C₈-Alkyl, vorzugsweise C₁-C₄-Alkyl, darunter vorzugsweise C₁-C₂-Alkyl wie Ethyl und vor allem Methyl;
C₂-C₈-Alkenyl, vorzugsweise C₂-C₆-Alkenyl, darunter vorzugsweise C₂-C₄-Alkenyl wie Propenyl, Butenyl und vor allem Ethenyl;
C₂-C₈-Alkinyl, vorzugsweise C₂-C₆-Alkinyl, darunter vorzugsweise C₂-C₄-Alkinyl wie Propinyl, Butinyl und vor allem Ethinyl;
C₃-C₈-Cycloalkyl, vorzugsweise C₅-C₇-Cycloalkyl wie Cyclopentyl, Cycloheptyl und besonders Cyclohexyl;
C₇-C₈-Arylalkyl, vorzugsweise Phenylethyl und ganz besonders Benzyl;
Aryl, vorzugsweise Phenyl.

Im Hinblick auf die gewünschten Verfahrensprodukte I ist das 2-Hydroxycyclohexanondimethylketal bzw. das 2-Methoxycyclohexanon besonders bevorzugt.

Der im erfindungsgemäßen Verfahren verwendete Katalysator ist Palladium auf Zirkonoxid als Trägermaterial.

Der Trägerkatalysator hat vorzugsweise einen Gehalt an aktiven Metallen von 0,2 bis 5, besonders 0,6 bis 1,5 Gew.-%, bezogen auf die Summe aus Träger und katalytisch aktivem Metall, berechnet als Metall.

Der erfindungsgemäße Katalysator besitzt eine BET-Oberfläche von 10 bis 300 m²/g Katalysator, bevorzugt 40 bis 150 m². Der mittlere Porendurchmesser liegt im Bereich von 2 bis 100 nm, bevorzugt im Bereich von 10 bis 30 nm.

Das Porenvolumen liegt im Bereich von 0,1 bis 1 ml/g Katalysator, bevorzugt im Bereich von 0,2 bis 0,5 ml/g Katalysator.

Die kristalline Modifikation des als Trägermaterial verwendeten Zirkonoxid ist zu einem Anteil von 60 bis 100%, bevorzugt 70 bis 90% monoklin.

Der Katalysator kann wahlweise als 2- bis 4-mm Stränge oder als Tabletten mit 3 bis 5 mm Durchmesser, als Splitt mit Teilchengrößen von 0,05 bis 1 mm, vorzugsweise 0,1 bis 0,5 mm als größten Durchmesser oder als Pulver mit Teilchengrößen von 0,05 bis 0,5 mm eingesetzt werden, wobei sich das Pulver auch als Wirbelkontakt eignet.

Die Katalysatorbelastung pro Stunde beträgt beim kontinuierlichen Verfahren 0,001 bis 1 kg, vorzugsweise 0,01 bis 0,5 kg der Verbindung II oder III pro kg des Katalysators.

Wirtschaftlich sehr vorteilhaft ist es, den Palladiumkatalysator in partiell desaktivierter Form einzusetzen, da dies zu höheren Produktselektivitäten führt.

Als Substanzen zur partiellen Vergiftung des Katalysators eignen sich: Schwefel-, Selen-, und/oder Tellur-haltige Verbindungen, Bariumsulfat, und Kohlenmonoxid die im allgemeinen in Mengen von 0,05 bis 0,3 Gew.-%, vorzugsweise 0,1 bis 0,15 Gew.-% der Verbindung II oder III eingesetzt werden oder dem Katalysator bei seiner Herstellung zugefügt werden.

Es kann zweckmäßig sein, zur Unterdrückung von Dehydratisierungsprozessen bei der Dehydrierung den Katalysator während der Präparation mit einer wäßrigen Lösung einer Base zu versehen. Dabei kann die wäßrige Lösung der Base z.B. durch Besprühen oder Tränken des Trägers aufgebracht werden. Bevorzugte Basen sind Alkalihydroxide wie Natron- oder Kalilauge, besonders bevorzugte Basen sind Alkalicarbonate wie Natriumcarbonat und vor allem Kaliumcarbonat.

Diese werden üblicherweise in Mengen von 50 bis 300, bevorzugt 100 bis 250 Gew.-%, bezogen auf Palladium eingesetzt.

Die Herstellung des zur Anwendung gelangenden Katalysators kann nach an sich bekannten Methoden erfolgen. So wird beispielsweise der Träger ZrO₂ mit Lösungen von Palladiumverbindungen, basischen Verbindungen und Desaktivatoren wie z.B. Schwefelverbindungen zusammen oder getrennt in beliebiger Reihenfolge behandelt. Das Trägermaterial kann dabei sowohl oberflächlich als auch durch und ; durch getränkt werden.

Die Überführung der Palladiumverbindung in die aktive metallische Form kann durch Reduktion in der Gasphase oder Flüssigphase oder durch thermische Behandlung erzielt werden. Zur Reduktion in der Gasphase können CO, CH₄ oder H₂ verwendet werden.

Der erfindungsgemäße Katalysator hebt sich von den bis dato bekannten Dehydrierkatalysatoren durch höher erzielbare Selektivitäten und längeren Standzeiten ab.

Üblicherweise führt man die Dehydrierung bei 200 bis 400°C, vorzugsweise bei 240 bis 340°C und ganz besonders bevorzugt bei 280 bis 300°C durch. Da in der Gasphase umgesetzt wird, muß die Temperatur mindestens so hoch gewählt werden, daß ein vollständiges verdampfen der Ausgangsverbindungen II oder III gewährleistet ist.

Aus verfahrenstechnischen Gründen empfiehlt es sich, die 2-Hydroxycyclohexanondialkylketale II bzw. die 2-Alkoxycyclohexanone III in Form von Lösungen einzusetzen und zusammen mit dem Lösungsmittel zu verdampfen. Bevorzugte Lösungsmittel zur Gewinnung der Verbindungen Ia sind Dioxan und Tetrahydrofuran. Besonders gute Ergebnisse erzielt man mit Methanol.

Zur Gewinnung der Verbindungen Ib ist Wasser, auch in Form von Gemischen mit den oben genannten Lösungsmitteln bevorzugt.

Im allgemeinen enthalten die Lösungen 10 bis 80 Gew.-%, bevorzugt 20 bis 50 Gew.-% der Verbindungen II oder III.

Zweckmäßigerweise wird die Umsetzung bei Normaldruck vorgenommen, jedoch kann man auch bei vermindertem oder leicht erhöhtem Druck arbeiten, also etwa im Bereich von 10 mbar bis 20 bar.

Die Verweilzeiten am Katalysator betragen normalerweise 0,1 bis 60 Sekunden, meistens 1 bis 5 Sekunden.

Die Umsetzung erfolgt in der Gasphase, wobei eine Festbettreaktion und eine Gasphasenreaktion im Wirbelbett in Betracht kommen.

Um die Katalysatoraktivität während der Dehydrierung aufrechtzuerhalten, empfiehlt es sich, ein Trägergas aus Wasserstoff oder einem Gemisch aus Wasserstoff und Stickstoff oder Argon zu verwenden.

Eine wirtschaftlich sehr vorteilhafte Ausführungsform des erfindungsgemäßen Verfahrens besteht in der Festbettreaktion, indem man das 2-Hydroxycyclohexanondialkylketal II bzw. das 2-Alkoxycyclohexanon III, gegebenenfalls in Lösung, verdampft und in der Gasphase, gegebenenfalls mit Hilfe eines Trägergases, über einen festen Katalysator leitet.

Man kann die Aufarbeitung der Reaktionsprodukte in an sich bekannter Weise vornehmen, und zwar in der Regel durch fraktionierte Destillation des Reaktionsgemisches und gegebenenfalls anschließende Extraktion.

Die nach dem erfindungsgemäßen Verfahren auf einfache und wirtschaftliche Weise erhältlichen Brenzcatechinmonoether Ia und Brenzcatechine Ib können als Zwischenprodukte zur Synthese von Pharmaprodukten wie Verapamil, oder von Riech- und Geschmacksstoffen wie Vanillin verwendet werden.

In den nachfolgenden Beispielen wird die erfindungsgemäße Verwendung des Dehydrierkatalysators Pd/ZrO₂ näher erläutert.

### Beispiel 1: Katalysatorherstellung

### Katalysator A:

1248,8 g ZrO₂-Tabletten (= 1168,87 g ZrO₂) wurden mit einer Lösung von 24,96 g Pd-Acetat (= 11,8 g Pd) und 40,7 g Oxalsäure in 500 ml Wasser besprüht und 16 h bei 95°C getrocknet. Nach Zugabe einer Lösung von 20,9 g K₂CO₃·1.5 H₂O (= 11,91 g K₂O) in 500 ml Wasser wurden die Tabletten erneut 48 h bei 95°C getrocknet. Im letzten Schritt wurden die Tabletten mit einer Lösung von 6,38 g 40%igem (NH₄)₂S (= 1,2 g S) in 510 ml Wasser versetzt und 24 h bei 100°C getrocknet.

Der fertige Katalysator hatte einen Palladium-Gehalt von 0,87 Gew.-%, einen Kalium-Gehalt von 0,81 Gew.-% und einen Schwefel-Gehalt von 0,09 Gew.-%. Die Palladiumteilchengröße betrug laut TEM (Transmissionselektronenmikroskopie) etwa 25 bis 150 nm, die Größe der ZrO₂-Teilchen 10 bis 20 nm. Die Gesamtporenfläche betrug laut Hg-Porosimetrie 92 m²/g, der mittlere Porendurchmesser 0,023 µm und das Gesamtporenvolumen 0,388 ml/g. Die BET-Oberfläche betrug 75 m²/g.

### Katalysator B:

757,9 g ZrO₂-Tabletten (= 730,62 g ZrO₂) wurden mit einer Lösung von Pd-Nitrat (= 7,38 g Pd) in 182 ml Wasser besprüht und 16 h bei 95°C getrocknet. Nach Zugabe einer Lösung von 13,09 g K₂CO₃·1,5 H₂O (= 7,46 g K₂O) in 162 ml Wasser wurden die Tabletten erneut 48 h bei 95°C getrocknet. Im letzten Schritt wurden die Tabletten mit einer Lösung von 3,99 g 40%igem (NH₄)₂S (= 0,75 g S) in 163 ml Wasser versetzt und 24 h bei 100°C getrocknet.

Der fertige Katalysator hatte einen Palladium-Gehalt von 0,93 Gew.-%, einen Kalium-Gehalt von 0,91 Gew.-% und einen Schwefel-Gehalt von 0,08 Gew.-%. Die Palladiumteilchengröße betrug laut TEM (Transmissionselektronenmikroskopie) etwa 3 bis 10 nm, die Größe der ZrO₂-Teilchen 3 bis 10 nm. Die Gesamtporenfläche betrug laut Hg-Porosimetrie 74 m²/g, der mittlere Porendurchmesser 0,014 µm und das Gesamtporenvolumen 0,228 ml/g.

### Katalysator C:

200 g ZrO₂-Stränge (BET-Oberfläche 92 m²/g) wurden mit einer Lösung von 4,27 g Pd-Acetat (= 2,025 g Pd) und 5,6 g Oxalsäure in 55 ml Wasser besprüht und 16 h bei 95°C getrocknet. Nach Zugabe einer Lösung von 3,61 g K₂CO₃·1,5 H₂O (= 2,06 g K₂O) in 55 ml Wasser wurden die Stränge erneut 48 h bei 95°C getrocknet. Im letzten Schritt wurden die Stränge mit einer Lösung von 1,1 g 40%igem (NH₄)₂S (= 0,207 g S) in 55 ml Wasser versetzt und 24 h bei 100°C getrocknet.

Der fertige Katalysator hatte einen Palladium-Gehalt von 0,97 Gew.-%, einen Kalium-Gehalt von 0,98 Gew.-% und einen Schwefel-Gehalt von 0,13 Gew.-%. Die Gesamtporenfläche betrug laut Hg-Porosimetrie 71 m²/g, der mittlere Porendurchmesser 0,013 µm und das Gesamtporenvolumen 0,23 ml/g.

### Katalysator D:

200 g ZrO₂-Stränge (BET-Oberfläche 70 m²/g) wurden mit einer Lösung von 4,27 g Pd-Acetat (= 2,025 g Pd) und 5,6 g Oxalsäure in 55 ml Wasser besprüht und 16 h bei 95°C getrocknet. Nach Zugabe einer Lösung von 3,61 g K₂CO₃·1,5 H₂O (= 2,06 g K₂O) in 55 ml Wasser wurden die Stränge erneut 48 h bei 95°C getrocknet. Im letzten Schritt wurden die Stränge mit einer Lösung von 1,1 g 40%igem (NH₄)₂S (= 0,207 g S) in 55 ml Wasser versetzt und 24 h bei 100°C getrocknet.

Der fertige Katalysator hatte einen Palladium-Gehalt von 0,98 Gew.-%, einen Kalium-Gehalt von 0,94 Gew.-% und einen Schwefel-Gehalt von 0,11 Gew.-%. Die Gesamtporenfläche betrug laut Hg-Porosimetrie 71 m²/g, der mittlere Porendurchmesser 0,017 µm und das Gesamtporenvolumen 0,29 ml/g.

### Katalysator E (Vergleichskatalysator gemäß DE-A-2 155 562: Beispiel 1):

Einer wäßrigen Lösung aus 300 g Ni(NO₃)₂·6H₂O, 75 g Cu(NO₃)₂·3H₂O, 9 g Cr(NO₃)₃·9H₂O, 0,6 g H₂SO₄, 0,35 g Na₂SO₄ wurden 1000 g Siliciumcarbid als Trägerstoff zugesetzt. Die erhaltene Mischung wurde unter Umrühren zum Abdampfen von Wasser erhitzt und anschließend 4 h in einem Luftstrom bei einer Temperatur von 400°C calciniert.

### Beispiel 2: Herstellung von Brenzcatechinmonomethylether aus Dimethoxycyclohexanol

Eine methanolische Lösung bestehend aus 6 g/h 2-Hydroxy-cyclohexanondimethylketal wurde bei einer Temperatur von 300°C verdampft und zusammen mit 60 l/h eines 1:1 Gemisches aus Wasserstoff und Stickstoff als Trägergas über die in Tabelle 1 genannten Katalysatoren geleitet. Die entstandenen Produkte wurden aufgefangen und gaschromatographisch analysiert. Die Standzeit der Katalysatoren wurde definiert als die Zeit, bis zu der die Selektivität auf 90% der maximal erreichten Selektivität gefallen ist.

**Tabelle 1:**

| Katalysator | Temperatur [°C] | Umsatz [%] | Selektivität [%] | WHSV⁺⁾ [h⁻¹] | Standzeit [h] |
|---|---|---|---|---|---|
| A | 300 | 100 | 86 | 0,03 | 122 |
| A | 300 | 95 | 80 | 0,19 | 25 |
| B | 300 | 100 | 85 | 0,02 | 73 |
| C | 300 | 100 | 83 | 0,02 | 298 |
| D | 300 | 100 | 82 | 0,02 | 186 |
| E (Vergleich) | 300 | 100 | 43 | 0,03 | 23 |
| F^{*)} (Vergleich) | 300 | 100 | 69 | 0,02 | 32 |
| G^{∇}) (Vergleich) | 300 | - | - | 0,02 | - |

| | | | | | |
|---|---|---|---|---|---|
| ⁺⁾ WHSV (weight hourly space velocity) = Katalysatorbelastung [g/g·h] | | | | | |
| ^{*)} Palladium auf Aktivkohle, gemäß EP-A-0 499 055; Beispiel 1 (Zusammensetzung: 1% Pd, 1,5% K₂CO₃, 0,1% S) | | | | | |
| ^{∇}) Palladium/Chrom auf Lithium-Aluminium-Spinell, gemäß DE 2064097 (Zusammensetzung: 2% Li, 0,66% Pd, 0.98% Cr). | | | | | |

### Beispiel 3: Herstellung von Brenzcatechinmonomethylether aus Methoxycyclohexanon

Eine methanolische Lösung bestehend aus 6 g/h 2-Methoxycyclohexanon wurde bei einer Temperatur von 300°C verdampft und zusammen mit 60 l/h eines 1:1 Gemisches aus Wasserstoff und Stickstoff als Trägergas über die in Tabelle 2 genannten Katalysatoren geleitet. Die entstandenen Produkte wurden aufgefangen und gaschromatographisch analysiert. Die Standzeit der Katalysatoren wurde definiert als die Zeit, bis zu der die Selektivität auf 90% der maximal erreichten Selektivität gefallen ist.

**Tabelle 2:**

| Katalysator | Temperatur [°C] | Umsatz [%] | Selektivität [%] | WHSV [h⁻¹] | Standzeit [h] |
|---|---|---|---|---|---|
| A | 300 | 100 | 90 | 0,07 | 372 |
| A | 300 | 93 | 88 | 0,12 | 47 |
| A | 300 | 97 | 85 | 0,22 | 21 |
| A | 300 | 86 | 88 | 0,31 | 27 |
| A | 300 | 99 | 82 | 0,46 | 10 |

### Beispiel 4: Herstellung von Brenzcatechin aus Methoxycyclohexanon

Eine analoge Durchführung wie in Beispiel 3, jedoch in wäßriger Lösung, führte in vergleichbaren Ausbeuten zu Brenzcatechin.

## Patentansprüche

1. Verfahren zur Herstellung von Brenzcatechinmonoethern oder Brenzcatechinen der allgemeinen Formel Ia oder Ib 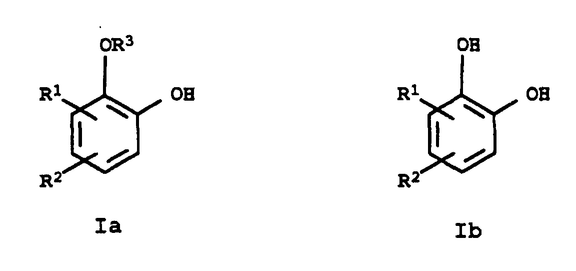 in denen R¹ und R² unabhängig voneinander für Wasserstoff oder C₁-C₈ Kohlenwasserstoffreste stehen und R³ einen C₁-C₈ Kohlenwasserstoffrest bedeutet, **dadurch gekennzeichnet, daß** man ein 2-Hydroxycyclohexanondialkylketal der allgemeinen Formel II oder ein 2-Alkoxycyclohexanon der Formel III 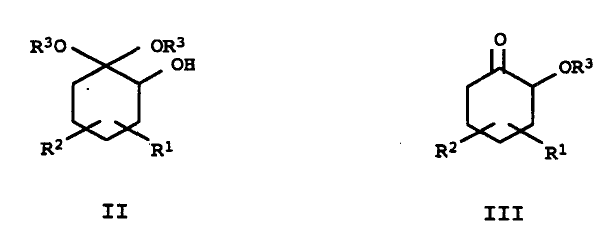
a) zur Gewinnung der Verbindungen Ia in Gegenwart von Palladium auf Zirkonoxid als Katalysator in der Gasphase umsetzt, wobei der Katalysator eine BET-Oberfläche von 10 bis 300 m²/g Katalysator und ein Porenvolumen im Bereich von 0,1 bis 1 ml/g Katalysator besitzt
oder
b) zur Gewinnung der Verbindungen Ib die Stufe a) in Gegenwart von Wasser durchführt.

2. Verfahren nach Anspruch 1, **dadurch gekennzeichnet, daß** der Katalysator eine BET-Oberfläche von 40 bis 150 m²/g Katalysator und ein Porenvolumen im Bereich von 0,2 bis 0,5 ml/g Katalysator besitzt.

3. Verfahren nach Anspruch 1, **dadurch gekennzeichnet, daß** die Reste R¹ und R² für Wasserstoff stehen und R³ eine Methylgruppe bedeutet.

4. Verfahren nach Anspruch 1, **dadurch gekennzeichnet, daß** man einen partiell desaktivierten Katalysator einsetzt.

5. Verfahren nach Anspruch 1, **dadurch gekennzeichnet, daß** die kristalline Modifikation des als Trägermaterial verwendeten Zirkonoxids zu einem Anteil von 60 bis 100% monoklin ist.

## Claims

1. A process for preparing catechol monoethers or catechols of the formula Ia or Ib 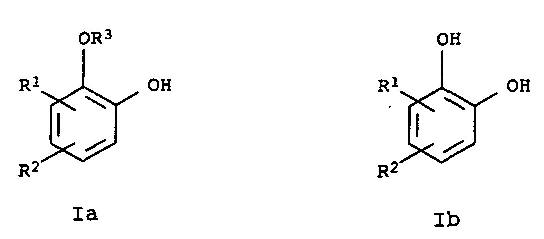 where R¹ and R² are, independently of one another, hydrogen or C₁-C₈ hydrocarbon radicals, and R³ is a C₁-C₈ hydrocarbon radical, which comprises a 2-hydroxycyclohexanone dialkyl ketal of the formula II or a 2-alkoxycyclohexanone of the formula III 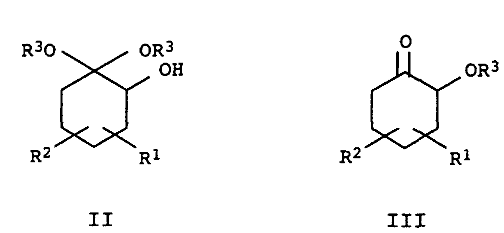
a) being reacted in the gas phase in the presence of palladium on zirconium oxide as catalyst to obtain compounds Ia, the catalyst having a BET surface area of from 10 to 300 m²/g of catalyst and a pore volume in the range from 0.1 to 1 ml/g of catalyst
or
b) carrying out stage a) in the presence of water to obtain compounds Ib.

2. A process as claimed in claim 1, wherein the catalyst has a BET surface area of from 40 to 150 m²/g of catalyst and a pore volume in the range from 0.2 to 0.5 ml/g of catalyst.

3. A process as claimed in claim 1, wherein R¹ and R² are each hydrogen, and R³ is methyl.

4. A process as claimed in claim 1, wherein a partially deactivated catalyst is employed.

5. A process as claimed in claim 1, wherein the crystal modification of the zirconium oxide used as carrier material is from 60 to 100% monoclinic.

## Revendications

1. Procédé pour la préparation de monoéthers de pyrocatéchols ou de pyrocatéchols de formules générales respectives Ia et Ib 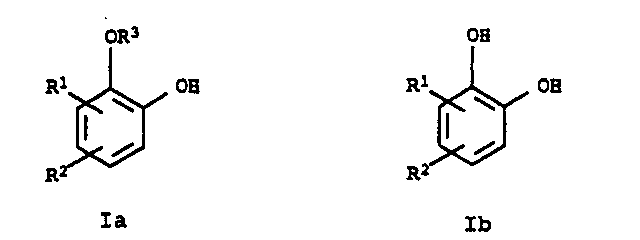 dans lesquelles R¹ et R² représentent chacun, indépendamment l'un de l'autre, l'hydrogène ou un radical hydrocarboné en C1-C8 et R³ représente un radical hydrocarboné en C1-C8, **caractérisé par le fait que** l'on soumet un dialkylacétal d'une 2-hydroxycyclohexanone de formule générale II ou une 2-alcoxycyclohexanone de formule III 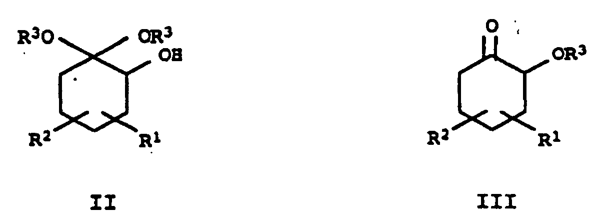
a) à conversion en phase gazeuse en présence de palladium sur oxyde de zirconium servant de catalyseur, le catalyseur ayant une surface BET de 10 à 300 m²/g du catalyseur et un volume de pores dans l'intervalle de 0,1 à 1 ml/g du catalyseur, pour obtenir les composés Ia
ou bien
b) en opérant comme décrit en a) ci-dessus mais en présence d'eau pour obtenir les composés Ib).

2. Procédé selon la revendication 1, **caractérisé par le fait que** le catalyseur a une surface BET de 40 à 150 m²/g du catalyseur et un volume de pores dans l'intervalle de 0,2 à 0,5 ml/g du catalyseur.

3. Procédé selon la revendication 1, **caractérisé par le fait que** les symboles R¹ et R² représentent l'hydrogène et R³ un groupe méthyle.

4. Procédé selon la revendication 1, **caractérisé par le fait que** l'on utilise un catalyseur partiellement désactivé.

5. Procédé selon la revendication 1, **caractérisé par le fait que** la forme cristalline de l'oxyde de zinc utilisé en tant que matière de support est monoclinique pour une proportion de 60 à 100 %.
